(19) **Europäisches Patentamt / European Patent Office / Office européen des brevets**

(11) **EP 4 481 753 A1**

(12) # EUROPEAN PATENT APPLICATION

(43) Date of publication:
**25.12.2024 Bulletin 2024/52**

(21) Application number: **23197321.5**

(22) Date of filing: **14.09.2023**

(51) International Patent Classification (IPC):
**G16H 20/30** (2018.01)     **A61B 5/00** (2006.01)
**G16H 40/20** (2018.01)     **G16H 50/30** (2018.01)
**A61B 5/0205** (2006.01)     **A61B 5/024** (2006.01)

(52) Cooperative Patent Classification (CPC):
**G16H 20/30; A61B 5/0002; A61B 5/6801;
A61B 5/7275; G16H 40/20; G16H 50/30;**
A61B 5/02055; A61B 5/02438; G16H 40/67

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **21.06.2023   US 202363509350 P**

(71) Applicant: **Koninklijke Philips N.V.
5656 AG Eindhoven (NL)**

(72) Inventors:
• **VERNOOIJ, Carlijn Andrea
  Eindhoven (NL)**

• **BONOMI, Alberto Giovanni
  Eindhoven (NL)**
• **DE GROOT, Koen Theo Johan
  5656AG Eindhoven (NL)**
• **VERHAEGH, Wilhelmus Franciscus Johannes
  Eindhoven (NL)**
• **EERIKÄINEN, Linda Maria
  Eindhoven (NL)**
• **HAAKMA, Reinder
  Eindhoven (NL)**
• **GELISSEN, Jozef Hubertus
  Eindhoven (NL)**

(74) Representative: **Philips Intellectual Property &
Standards
High Tech Campus 52
5656 AG Eindhoven (NL)**

(54) **SENSOR-BASED TREATMENT SCHEDULING**

(57)     A central control system includes a receiver, a memory and a processor. The receiver receives, from a plurality of wearable sensing systems, characteristic data determined to reflect health of wearers of the wearable sensing systems. The memory stores instructions. The processor executes the instructions. When the processor executes the instructions, the central control system is configured to: determine when the characteristic data indicates that any of the wearers of the wearable sensing systems is ready to be scheduled for a treatment, and initiate a process to schedule the treatment.

FIG.3

EP 4 481 753 A1

**Description**

BACKGROUND OF THE INVENTION

Vital signs monitoring typically includes monitoring one or more of the following physical parameters: heart rate, blood pressure, respiratory rate, core body temperature and blood oxygenation (SpO2). To assess a patient's condition or to determine a patient's degree of illness, often a clinical decision support algorithm is being used, e.g., the spot-check-based early warning score (EWS) system in the general ward. These algorithms take (a subset of) vital signs signals measured of a patient and analyze trends or compare values to a predefined threshold of assumed normal values. The output of these algorithms informs clinicians on the assumed physiological instability of the patient based on which, combined with non-physiological factors, the clinician can assess whether changes are needed in the patient's treatment plan. While these algorithms are aimed at instability detection, they are sometimes used as indirect indicator of patient stability, and patient stability algorithms are also being developed. With the introduction of unobtrusive wearable vital signs monitoring systems such as vital sign patches, continuous collection of vital signs is made feasible in lower acuity care settings like the general ward and at home. This opens the door for innovation, e.g., developing an early warning system based on the continuous measurements of a subset of the vital signs that are measured as spot-check in a conventional early warning system. Additionally, one can now think of use cases benefitting from clinical decision support algorithms for patients wearing the vital signs monitors at home.

As hospital systems need to keep a tight financial balance, hospital systems need the best estimation possible for planning treatments. Seeing as the largest cost of hospital systems is staff members, hospital systems need to use staff members as efficient as possible. While clinical decision support algorithms may define whether a patient's treatment should be *modified,* these types of algorithms are not employed to define whether a patient should *start* a treatment. Such an estimation of a fitness of a patient to start treatment could support hospital critical planning like increased same day surgeries, decreased surgical cancellations, improved discharge readiness, decreased complications after treatment start, etc. While there are programs to get patients prepared for surgery as well as possible, and to make them fit for surgery, there is no assessment to objectively evaluate their fitness.

P atient fitness for treatment is currently estimated by clinical staff. Cardiovascular exercise tests may give an objective measurement of patient fitness. However, executing cardiovascular exercise tests is impractical, costly, and sometimes even impossible due to patient limitations. Moreover, patient fitness is not necessarily properly assessed solely on the basis of a fitness index score resulting from a questionnaire.

SUMMARY OF THE INVENTION

According to an aspect of the present disclosure, a central control system includes a receiver, a memory and a processor. The receiver receives, from a plurality of wearable sensing systems, characteristic data determined to reflect health of wearers of the wearable sensing systems. The memory stores instructions. The processor executes the instructions. When the processor executes the instructions, the central control system is configured to: determine when the characteristic data indicates that any of the wearers of the wearable sensing systems is ready to be scheduled for a treatment, and initiate a process to schedule the treatment.

According to another aspect of the present disclosure, a sensing system includes a sensor, a control system, and a wireless communication unit. The sensor senses characteristic data determined to reflect health of a user of the sensing system. The control system includes a memory that stores instructions and a processor that executes the instructions. When the processor executes the instructions, the control system is configured to determine when the characteristic data indicates that the user is ready to be scheduled for a treatment. The wireless communication unit transmits a wireless notification to initiate a process to schedule the treatment.

According to another aspect of the present disclosure, an operation method for a central control system includes receiving from a plurality of wearable sensing systems, characteristic data determined to reflect health of wearers of the wearable sensing systems; obtaining instructions by a processor from a memory; determining, based on the processor executing the instructions, when the characteristic data indicates that any of the wearers of the wearable sensing systems is ready to be scheduled for a treatment, and initiating a process to schedule the treatment.

According to another aspect of the present disclosure, an operation method of a wearable sensing system includes sensing, by a sensor, characteristic data determined to reflect health of a wearer of the wearable sensing system; obtaining instructions by a processor from a memory; determining, by a control system that includes the processor and the memory, when the characteristic data indicates that the wearer is ready to be scheduled for a treatment, and transmitting, by a wireless communication unit, a wireless notification to initiate a process to schedule the treatment.

BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.

Fig. 1 illustrates a system for sensor-based treatment scheduling, in accordance with a representative embodiment.
Fig. 2 illustrates another system for sensor-based treatment scheduling, in accordance with a representative embodiment.
Fig. 3 illustrates another system for sensor-based treatment scheduling, in accordance with a representative embodiment.
Fig. 4A illustrates a method for sensor-based treatment scheduling, in accordance with a representative embodiment.
Fig. 4B illustrates another method for sensor-based treatment scheduling, in accordance with a representative embodiment.
Fig. 5 illustrates a first visualization of patient fitness over time, in accordance with a representative embodiment.
Fig. 6 illustrates a second visualization of patient fitness over time, in accordance with a representative embodiment.
Fig. 7 illustrates a method for sensor-based treatment scheduling, in accordance with a representative embodiment.
Fig. 8 illustrates a computer system, on which a method for sensor-based treatment scheduling is implemented, in accordance with another representative embodiment.

DETAILED DESCRIPTION OF EMBODIMENTS

In the following detailed description, for the purposes of explanation and not limitation, representative embodiments disclosing specific details are set forth in order to provide a thorough understanding of embodiments according to the present teachings. However, other embodiments consistent with the present disclosure that depart from specific details disclosed herein remain within the scope of the appended claims. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. Definitions and explanations for terms herein are in addition to the technical and scientific meanings of the terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

As used in the specification and appended claims, the singular forms of terms 'a', 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to", "coupled to", or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below.

As described herein, the advent of health-related unobtrusive sensing systems may enable a shift from conventional hospital treatment by replacing conventional hospital treatment with unobtrusive vital signs sensor technologies, centered around the individual, to provide better information about a patient's general health condition. Such vital signs sensor technologies may reduce treatment costs by disease prevention and enhance the quality of life and, potentially, improve physiological data for physicians to analyze when attempting to diagnose a patient's general health condition. Algorithms described herein may estimate treatment readiness of patients and help hospitals scheduling surgeries/treatments to decrease surgery cancellations, increase same day surgery, estimate discharge readiness, start treatment whenever possible by indicating patient decompensation possibilities/patient physical status instead of guessing/basing estimations on relatively few metrics. The teachings herein describe employment of clinical decision support algorithms to define a patient's fitness to start a treatment based on unobtrusively measured vital signs, such as for fitness for surgery, fitness for intervention, and/or fitness for discharge. Based on these fitness estimations, patient selection for treatment may be improved. The sensor-based treatment scheduling may be implemented, e.g., for outpatient monitoring, remote patient monitoring, and in-hospital patient monitoring.

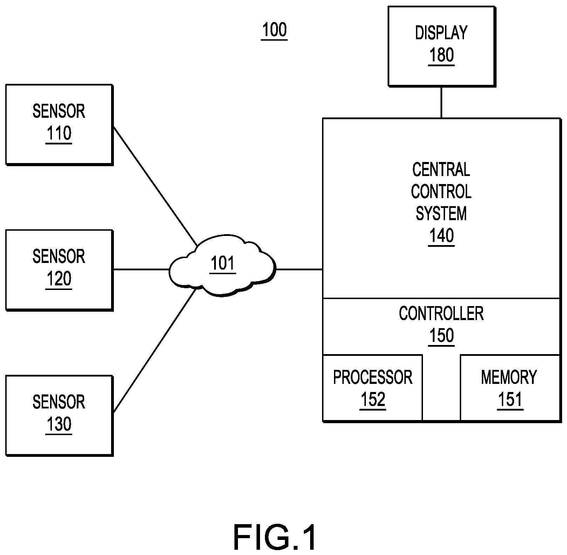

Fig. 1 illustrates a system 100 for sensor-based treatment scheduling, in accordance with a representative embodiment.

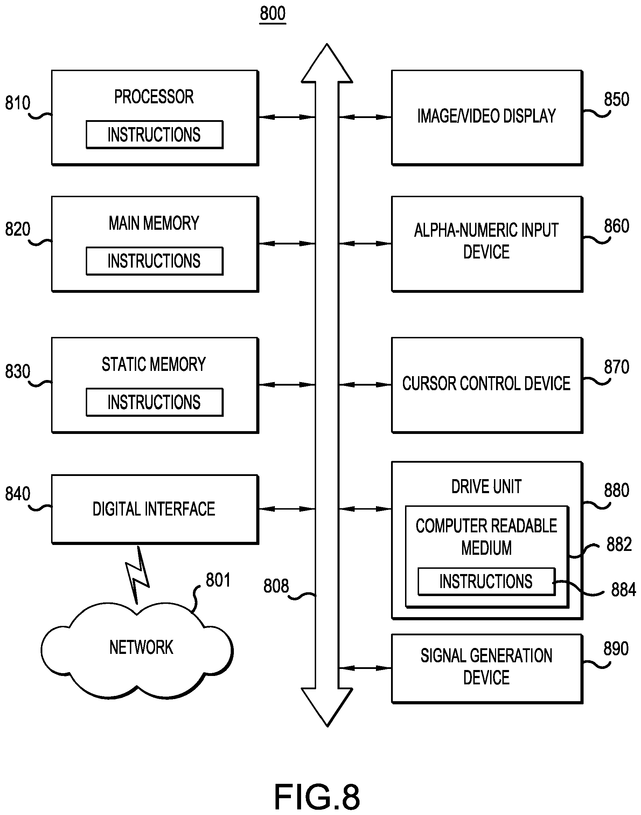

The system 100 in Fig. 1 is a system for sensor-based treatment scheduling and includes components that are distributed. In Fig. 1, most aspects of logical operations described herein are performed by the central control system 140 using the controller 150. The system 100 includes a first sensor 110, a second sensor 120, a third sensor 130, a network 101, and a central control system 140. The central control system 140 includes a controller 150, and the controller 150 includes at least a memory 151 that stores instructions and a processor 152 that executes the instructions. A computer that can be used to implement the controller 150 is depicted in Fig. 8, though a controller 150 may include more or fewer elements than depicted in Fig. 1 or Fig. 8.

Examples of the sensors in Fig. 1 include medical wearable devices, torso-worn vital signs health patch, wrist-worn (multi color) photoplethysmography (PPG) watches, sensors equipped in chest straps, smart garments, medical ear buds/over the ear, forehead/nose sensors, and medical vital signs camera. Sensors described herein may also include non-wearable sensors such as electronic scales. The central control system 140 may include a receiver that receives, from a plurality of wearable sensing systems, characteristic data determined to reflect health of wearers of the wearable sensing systems.

The system 100 in Fig. 1 may be used to assess and predict patient fitness for treatment using an algorithm which is based on continuous signals generated by a vital signs monitor system. One benefit of using a central control system 140 to make the assessments and predictions, is that the central control system 140 may, when appropriate, balance the resources of a facility and the requirements for multiple patients. In other words, scheduling may be customized for a patient based on sensor readings for the patient, while also being balanced with resources and competing needs of other patients when appropriate. Moreover, the scheduling described herein is not limited to any particular class of treatments, and instead may be used for a variety of types of treatments including cancer patients, cardiac patients, respiratory patients, and patients with other types of maladies.

A vital signs monitor may include any one or more of the first sensor 110, the second sensor 120, and/or the third sensor 130. Commercially available vital signs monitors such as patches and smartwatches and mobile phones may be used to embed patient fitness software, such that the patches, smartwatches and mobile phones may serve as any instance of the first sensor 110, the second sensor 120 and/or the third sensor 130. Other than patient stability/instability algorithms, a main parameter included in the patient fitness estimation is patient activity. The algorithm assesses patient fitness and provides a means to prioritize and select patients ready for treatment.

In some embodiments, the first sensor 110, the second sensor 120 and/or the third sensor may comprise a wearable sensor used to collect patient data to derive an indication about the fitness for treatment level and the temporal progression of the fitness level from the last moment of care which could be a hospital visit or a phone consultation. The wearable sensor may be equipped with an accelerometer, a gyroscope, a PPG-sensor, and ECG-sensor or a similar sensor or sensor-combination in which body movements and physiological modalities are measured. The measurements of body movements and physiological modalities may produce information which can be used to characterize patient fitness. Examples of such information may include the number of steps per day, the energy expenditure, the oxygen consumption over activity or an aggregate indicator of body movement and posture in relation to physiological modalities like heart rate and respiration rate are examples of the fitness indicators obtained from the wearable sensor.

The controller 150 may include interfaces, such as a first interface, a second interface, a third interface, and a fourth interface. One or more of the interfaces may include ports, disk drives, wireless antennas, or other types of receiver circuitry that connect the controller 150 to other electronic elements. One or more of the interfaces may also include user interfaces such as buttons, keys, a mouse, a microphone, a speaker, a display separate from the display 180, or other elements that users can use to interact with the controller 150 such as to enter instructions and receive output. The interface(s) of the controller 150 may include a receiver that receives, from a plurality of the above-described sensors, characteristic data determined to reflect health of wearers of the wearable sensing systems. Any particular wearer may wear one or more wearable sensing system(s), and/or may otherwise use one or more non-wearable sensing system(s), and the sensing systems may generate and send the characteristic data to the controller 150 of the central control system 140.

The controller 150 may perform some of the operations described herein directly and may implement other operations described herein indirectly. For example, the controller 150 may indirectly control operations such as by generating and transmitting content to be displayed on the display 180. The controller 150 may directly control other operations such as logical operations performed by the processor 152 executing instructions from the memory 151 based on input received from electronic elements and/or users via the interfaces. Accordingly, the processes implemented by the controller 150 when the processor 152 executes instructions from the memory 151 may include steps not directly performed by the controller 150.

The central control system 140 may be used to implement a decision support system designed to process fitness level data and advise clinical practitioners on the appropriateness of a specific treatment prescription for an individual. The decision support system implemented by the central control system 140 may evaluate the trends in fitness index over time to identify when a patient is still, when a patient is likely to be fighting off an infection, when a patient's health is deteriorating, and when a patient became or is no longer eligible for a treatment which is likely to not bring health benefits. The central control system 140 may be configured to determine that characteristic data reflected in trends evaluated over time indicates that the wearer of a wearable sensing system is ready to be scheduled for a treatment based on evaluations of trends of the characteristic data. The characteristic data described herein is determined to reflect health of a wearer or other user of a sensor. A fitness index derived from wearable sensors data from the first sensor 110, the second sensor 120 and/or the third sensor 130 may also be compared to a distribution of population values of the fitness levels in order to create a cross-sectional view on the patient population and select from the cohort the patients with the most optimal fitness level for a specific treatment.

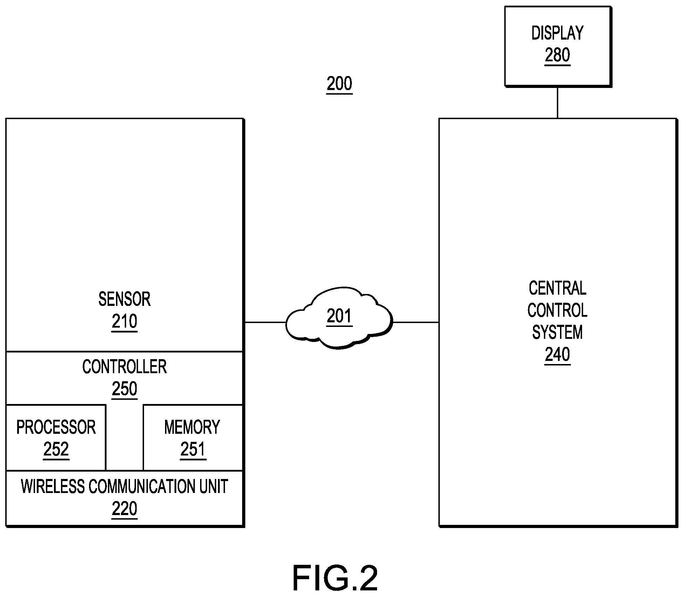

Fig. 2 illustrates another system for sensor-based treatment scheduling, in accordance with a representative embodiment.

The system 200 in Fig. 2 includes a sensor 210 a network 201, a central control system 240, and a display 280. In Fig. 2, most aspects of logical operations described herein are performed by the sensor 210 using the controller 250. The sensor 210 includes a controller 250 and a wireless communication unit 220. The controller 250 includes a memory 251 that stores instructions and a processor 252 that executes the instructions. The controller 250 may also include interfaces, such as a first interface, a second interface, a third interface, and a fourth interface. Although the system 200 is shown to include a wireless communication unit 220, in some embodiments a sensor may also or alternatively include a wired communication unit, such as an interface for an ethernet cable.

The network 201 may comprise one or more wired and/or wireless networks. For example, the network 201 may include a WiFi network, a wide area wired network, and/or any other types of networks that may be used to connect a wireless device such as the sensor 210 to a central control system 240 over the internet.

The wireless communication unit 220 may comprise an application-specific integrated circuit configured to wirelessly send characteristic data from the sensor 210 and to wirelessly receive instructions and other types of data from the central control system 240.

The controller 250 may perform some of the operations described herein directly and may implement other operations described herein indirectly. For example, the controller 250 may directly control operations such as logical operations performed by the processor 252 executing instructions from the memory 251 based on characteristic data generated by the sensor 210.

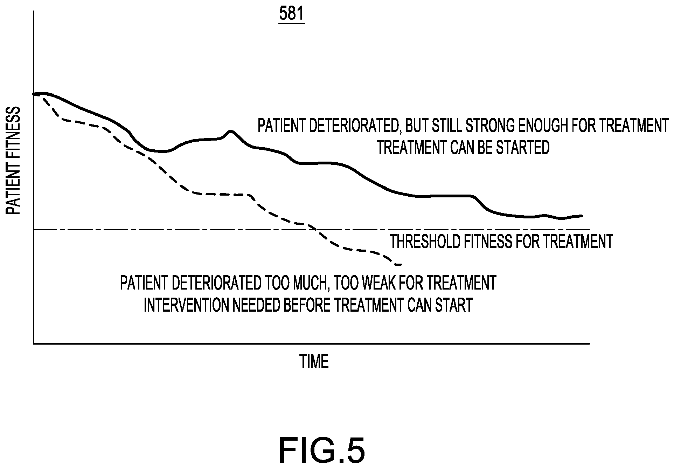

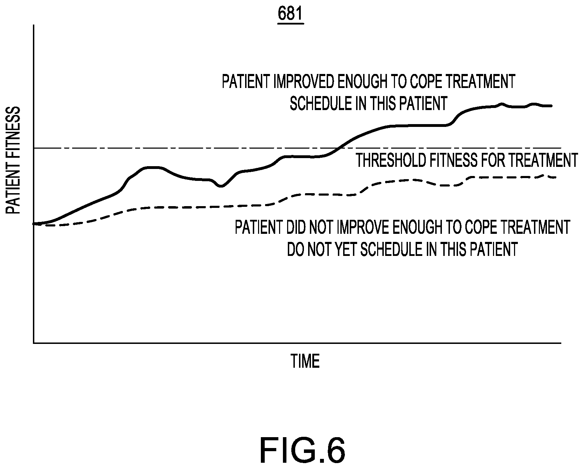

The display 280 may be used to display user interfaces such as those shown in and described with respect to Fig. 5 and Fig. 6 below.

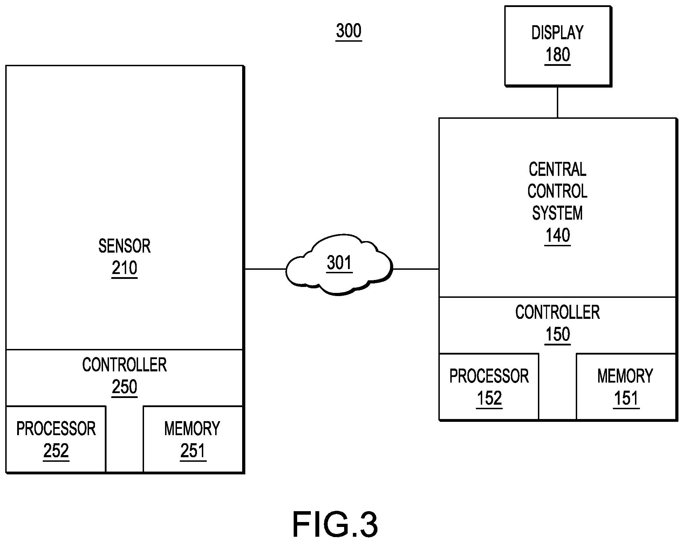

Fig. 3 illustrates another system for sensor-based treatment scheduling, in accordance with a representative embodiment.

The system in Fig. 3 includes a sensor 210, a network 301, the central control system 140, and the display 180. The sensor 210 includes the controller 250 with the memory 251 and the processor 252. The central control system 140 includes the controller 150 with the memory 151 and the processor 152. In Fig. 3, aspects of logical operations described herein are distributed between the sensor 210 using the controller 250 and the central control system 140 using the controller 150. For example, the sensor 210 may make a preliminary determination when a treatment should be scheduled, and the central control system 140 may balance the needs of multiple patients to optimize the scheduling for the multiple patients as a whole.

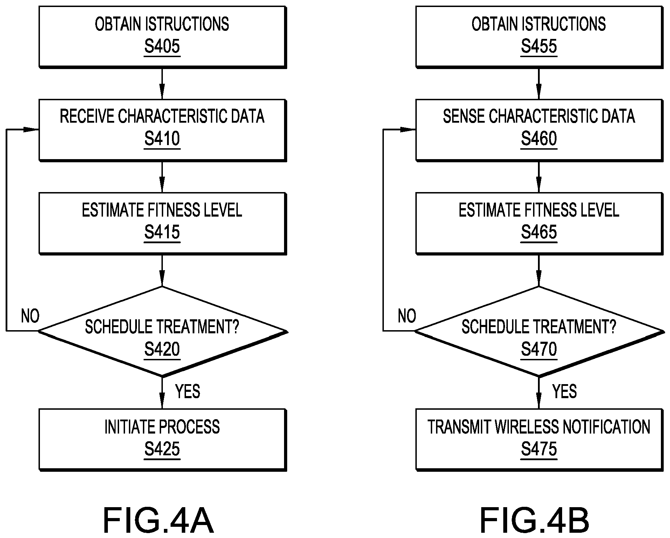

Fig. 4A illustrates a method for sensor-based treatment scheduling, in accordance with a representative embodiment.

The method of Fig. 4A may be performed by any of the facility systems or other centralized systems described herein, based on characteristic data received from any of the sensors described herein.

At S405, instructions are obtained. The instructions obtained at S405 may be instructions retrieved or otherwise received for how to process characteristic data at the central control system 140 or the central control system 240. For example, the instructions may pertain to how to process characteristic data from different sensors of different types from different sources. In some embodiments, the central control system 140 and/or the central control system 240 may be dedicated to processing characteristic data for one or more specific types of medical concerns. In other embodiments, the central control system 140 and/or the central control system 240 may be dedicated as general-purpose sensor-based treatment schedulers, such as for a hospital.

At S410, characteristic data is received. The characteristic data may be received from any of the sensors described herein, and may be received in a specific format used for the sensor-based treatment scheduling described herein. For example, a format may specify a type of sensor, a patient or other subject using the sensor, a type of characteristic data being received, and the actual readings sensed by the sensor and included in the characteristic data. A format may also include date and time.

To be sure, characteristic data may be received thousands of times a minute from various sensors by the central control system 140 or the central control system 240. The central control system 140 and/or the central control system 240 may be programmed to systematically process such characteristic data in the manner described herein, particularly insofar as the characteristic data may reflect an urgent need to immediately schedule a treatment in some cases.

At S415, a fitness level is estimated. The fitness level may be estimated by applying an algorithm to the characteristic data received at S410. The algorithm applied to estimate a fitness level may estimate a fitness level of a wearer of a wearable sensing system. The algorithm may comprise a function that maps characteristic data received over time to a fitness level score, and determine when the fitness level score for the wearer of the wearable sensing system exceeds a threshold.

The fitness level estimation at S415 may also take into account environmental data. For example, temperature, humidity and pollen levels may affect some respiratory conditions, and changes in environmental conditions may be taken into account when determining the fitness level. As one particular example, expectations for elevated pollution levels may be used as a basis to expedite a treatment schedule for a respiratory patient.

The function that maps characteristic data received over time to a fitness level score may be a function that weights sums based on the characteristic data received over time. In some embodiments, the function that maps characteristic data to a fitness level score may comprise a pre-trained machine learning model which infers the fitness level score from the characteristic data. At least one of the weighted sums comprises or may comprise a weighted sum based on activity of the wearer of the wearable sensing system. A basic example calculation of patient fitness for treatment is illustrated by equation (1) below:

$$(1)$$

$$F4T(t) = w_1 \frac{1}{N} \sum_{s=t-N+1}^{t} Act(s) + w_2 \frac{1}{N} \sum_{s=t-N+1}^{t} \frac{HR(s)}{Act(s)}$$

In equation (1) above:

t,s equals time,
N equals the length averaging time window,
w1, w2 equal a weighting factor, which may be set by a table or another equation, and which may be based on, e.g., demographics, body mass index (BMI), relative active and resting heart rate, relative activity level, increasing or decreasing activity over time, changes in heart rate relative to increasing or decreasing activity over time, or a combination thereof.
Act(s) equals activity at time s, and
HR(s) equals heart rate at time s.

With w1 > 0, a higher average activity may give a higher fitness score. With w2 < 0, a higher heart rate may result in a lower fitness score, unless the higher heart rate corresponds to a higher activity level. Optionally, the activity level may be squared, or another non-linear transformation may be applied, to value higher activity levels increasingly more. The averaging time window of length N may be used to eliminate outliers in data, but averaging the characteristic data over a running time period such as 5 minutes or 1 hour.

The basic model from equation (1) may be expanded with more features in an embodiment set by equation (2) as follows:

$$(2)\ F4T(t) = w_1 \frac{1}{N} \sum_{s=t-N+1}^{t} Act(s) + w_2 \frac{1}{N} \sum_{s=t-N+1}^{t} \frac{HR(s)}{Act(s)} +$$

$$w_3 \frac{1}{N} \sum_{s=t-N+1}^{t} \frac{RR(s)}{Act(s)} + w_4 \frac{1}{N} \sum_{s=t-N+1}^{t} CBT(s) + w_5 CR(t) + int(t) + ext(t)$$

In equation (2) above:

CR equals a circadian rhythm measurement,

CBT equals a core body temperature parameter,

RR equals respiration rate

int equals an internal factors index, which may be based on e.g. lifestyle, comorbidities, age, smoking, nutrition, alcohol consumption, psychological state etc, and may be set by another equation,

ext equals an external factors index, which may be based on e.g. seasonal affects (e.g. flu, allergies, seasonal affective disorder), external temperature and humidity, psychological environment (e.g. social stress), etc, and may be set by another equation.

Using equation (2) above, a central control system may be configured to add an internal factor index int to the weighted sums for the wearer of a wearable sensing system. The internal factor index int may comprise a first value determined based on demographic and lifestyle characteristics of the wearer of the wearable sensing system. A circadian/regularity factor may also be taken into account. Using equation (2) above, a central control system may also be configured to add an external factor index ext to the weighted sums for the wearer of the wearable sensing system. The external factor index ext may comprise a second value based on health characteristics of the wearer of the wearable sensing system. If the patient shows a regular circadian activity rhythm, the algorithm may be higher than if the patient shows periods of relatively high activity during the night or low levels of activity during the day. If the patient has a regular circadian core body temperature rhythm, the patient algorithm may be higher than if the patient shows periods of relatively high core body temperature during the night or low levels of core body temperature during the day. If the patient has a low heart rate and respiratory rate during the night, the patient algorithm may be higher than if the patient shows periods of relatively high heart rate or respiratory rate during the night or low levels of heart rate or respiratory rate during the day.

Internal and external factors may also influence a patient's (future) level from the algorithm. Internal factors include patient characteristics such as age and body mass index, but also comorbidities, as well as lifestyle characteristics like smoking, alcohol consumption etc. External factors may include environmental and contextual factors such as seasonal effects like hay fever. The more of these internal and external factors negatively influence the patient fit4treatment, the higher the internal and external factor index and the lower the fit4treatment level. These factors can be used for current fit4treatment estimations, but also for indicative future fit4treatment levels, e.g. in the case of hay fever where the patient might get gradually worse once spring starts.

At S420, a determination is made whether to schedule treatment. If a treatment is not to be scheduled (S420 = No), the process returns to S410. Of course, when the method of Fig. 4A is being performed by the central control system 140 or the central control system 240, the method may be run continuously as characteristic data is received from different sensors for different subjects. Therefore, the process may return to S410 even when a treatment is to be scheduled. If a treatment is to be scheduled (S420 = Yes), at S425, a process is initiated to schedule the treatment. The process to schedule the treatment may involve automatically sending a notification to a scheduling system over an electronic communication network specifying the subject, the treatment to be scheduled, and a relative urgency of scheduling the treatment. The treatment may involve a surgery, a shot, a therapy, a counseling session, or any other type of treatment for which scheduling may be optimized for patients.

Fig. 4B illustrates another method for sensor-based treatment scheduling, in accordance with a representative embodiment.

The method of Fig. 4B may be performed by any of the sensors described herein.

At S455, instructions are obtained. The instructions may be obtained when the first sensor 110 is programmed by placing instructions in the memory 151 to be executed by the processor 152. The instructions may be configured to logically process data sensed by the first sensor 110, and to determine when to transmit a wireless notification when a treatment is to be scheduled.

At S460, characteristic data is sensed. The characteristic data may be sensed over time by any one or more of the first sensor 110, the second sensor 120 and/or the third sensor 130. The characteristic data may be for physiological characteristics of a subject, such as weight, heart rate, temperature, blood pressure, or any of the other types of physiological characteristics which sensors are configured to sense.

At S465, a fitness level is estimated. The fitness level may be estimated by applying an algorithm to the characteristic data received at S460. The fitness level may be estimated by applying an algorithm to the characteristic data received at S410. The algorithm applied to estimate a fitness level may estimate a fitness level of a wearer of a wearable sensing system. The algorithm may comprise a function that maps characteristic data received over time to a fitness level score, and determine when the fitness level score for the wearer of the wearable sensing system exceeds a threshold. The explanation of such an algorithm is provided above in relation to S415, and is not repeated here for the sake of brevity. However, notably for the method of Fig. 4B, the algorithm is run by the first sensor 110, the second sensor 120 or the third sensor 130, rather than the central control system 140 or the central control system 240.

At S470, a determination is made whether to schedule a treatment. If a determination is made not to schedule a treatment (S470 = No), the method of Fig. 4B returns to S460. If a treatment is to be scheduled (S470 = Yes), at S475, a wireless notification is transmitted. Similar to the method of Fig. 4A, although S470 is shown as a binary determination, a determination to schedule a treatment may result in both outcomes from S470, i.e., to transmit the wireless notification and to continue sensing characteristic data. For example, characteristic data may continue to be sensed in case the characteristic data makes the scheduling of treatment more urgent.

Fig. 5 illustrates a first visualization of patient fitness over time, in accordance with a representative embodiment.

In Fig. 5, patient fitness is charted overtime. The patient fitness may be determined by a sensor or a central control system based on characteristic data sensed by one or more sensors. The visualization in Fig. 5 may be displayed via a user interface 581, such as on the display 280 in Fig. 2. Derived information on an individual patient fitness level and the relationship between the patient fitness and the population fitness may be displayed on a clinical dashboard for further visualization and decision making by a care team. In Fig. 5, the first visualization shows patient fitness that is trending down over time. Fig. 5 includes a threshold fitness for treatment, so that when the patient fitness shown by the dashed lines crosses below the threshold fitness for treatment, a process is initiated at S425 or a wireless notification is transmitted at S475. The patient fitness shown by the solid line does not cross below the threshold fitness for treatment, so no scheduling process is initiated and no wireless notification is sent based on the data reflected in the solid line. As an example implementation of the first visualization in Fig. 5, the central control system 140 or the central control system 240 may be configured to determine that characteristic data reflected in trends evaluated over time indicates that the wearer of a wearable sensing system is ready to be scheduled for a treatment based on evaluations of trends of the characteristic data, such as based on a decreasing trend over time as shown in Fig. 5.

Fig. 6 illustrates a second visualization of patient fitness over time, in accordance with a representative embodiment.

The visualization in Fig. 6 may be displayed via a user interface 681, such as on the display 280 in Fig. 2. In Fig. 6, the second visualization shows patient fitness that is trending up over time. Based on the dashed line in Fig. 6, a patient has not shown enough improvement to initiate a scheduling request. For example, sensors may be used to determine when a patient has recovered enough to schedule another appointment or treatment, and the absence of improvement may be used to delay an appointment or treatment. The solid line in Fig. 6 is for a patient who has improved enough to initiate an appointment or a follow-up treatment.

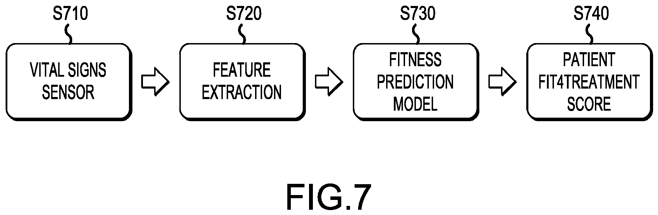

Fig. 7 illustrates a method for sensor-based treatment scheduling, in accordance with a representative embodiment.

In Fig. 7, feature extraction is incorporated into the sensor-based treatment scheduling.

At S710, a vital signs sensor senses vital signs. The vital signs sensor may include a pulses per revolution (PPR) sensor, an accelerometer, a temperature sensor, an electrical sensor, a light sensor, or another type of sensor.

At S720, a feature extraction block extracts features. A feature extraction block may be implemented by a processor/memory combination or by an application-specific integrated circuit (ASIC) dedicated to extracting features from physiological characteristics of a subject sensed by a sensor. Extracted features may include activity, heart rate, respiration rate, circadian rhythm, internal factors, external factors are extracted from the various sensors. Extracted features may also include combinations of features, such as the relation between heart rate and activity, the relationship between internal/external factors and HR, the relationship between internal/external factors and activity. A relation between heart rate and activity may comprise a ratio or a correlation over a specified time window, or heart rate recovery. A relationship between internal/external factors and heart rate may comprise a ratio between age and heart rate. A relationship between internal/external factors and activity may comprise a ratio between age and activity level.

At S730, a fitness prediction model may predict fitness. The fitness prediction model may map the combined extracted features onto a fitness score, e.g. using a formula as shown above, or using a pre-trained machine learning model such as a logistic regression model, a decision tree, a random forest model, a support vector machine or a neural network. The pre-trained machine learning model may infer a fitness level score from characteristic data generated by a sensor. The predicted fitness may be a numerical score, and may be specifically used for a particular type of condition for which patient fitness is being monitored. In some embodiments, more than one fitness score may be predicted, such as for different types of conditions.

At S740, a patient fit4treatment score may be produced by a patient algorithm as a final score. The final score may comprise a value specifically used for initiating a process or sending a wireless notification as described herein.

In some embodiments based on a modification to Fig. 7, an expanded set of more detailed features may be incorporated. For example, at S720, the feature extraction block may take into consideration acceleration, heart rate, circadian rhythm, internal factors, external factors, the relation between heart rate and activity, the relationship between internal/external factors and heart rate, the relationship between internal/external factors and activity, etc.

In still other embodiments based on more or different modifications to Fig. 7, a pre-trained machine learning model may be used for the fitness prediction model at S730. A pre-trained machine learning model may include a logistic regression model, a decision tree model, neural network models, or other types of models. The machine learning model is configured/trained with the task to provide a prediction of the current/instantaneous fitness level which is compared to a threshold to assess readiness for treatment. The machine learning model is also configured/trained to provide a form of forecast of time until a required fitness level is achieved. The forecast may range from 0 #days to an infinite number of days.

Fig. 8 illustrates a computer system, on which a method for sensor-based treatment scheduling is implemented, in accordance with another representative embodiment.

Referring to Fig. 8, the computer system 800 includes a set of software instructions that can be executed to cause the computer system 800 to perform any of the methods or computer-based functions disclosed herein. The computer system 800 may operate as a standalone device or may be connected, for example, using a network 801, to other computer systems or peripheral devices. In embodiments, a computer system 800 performs logical processing based on digital signals received via an analog-to-digital converter.

In a networked deployment, the computer system 800 operates in the capacity of a server or as a client user computer in a server-client user network environment, or as a peer computer system in a peer-to-peer (or distributed) network environment. The computer system 800 can also be implemented as or incorporated into various devices, such as a sensor, a central control computer, a workstation that includes a controller, a stationary computer, a mobile computer, a personal computer (PC), a laptop computer, a tablet computer, or any other machine capable of executing a set of software instructions (sequential or otherwise) that specify actions to be taken by that machine. The computer system 800 can be incorporated as or in a device that in turn is in an integrated system that includes additional devices. In an embodiment, the computer system 800 can be implemented using electronic devices that provide voice, video or data communication. Further, while the computer system 800 is illustrated in the singular, the term "system" shall also be taken to include any collection of systems or sub-systems that individually or jointly execute a set, or multiple sets, of software instructions to perform one or more computer functions.

As illustrated in Fig. 8, the computer system 800 includes a processor 810. The processor 810 may be considered a representative example of a processor of a controller and executes instructions to implement some or all aspects of methods and processes described herein. The processor 810 is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor 810 is an article of manufacture and/or a machine component. The processor 810 is configured to execute software instructions to perform functions as described in the various embodiments herein. The processor 810 may be a general-purpose processor or may be part of an application specific integrated circuit (ASIC). The processor 810 may also be a microprocessor, a microcomputer, a processor chip, a controller, a microcontroller, a digital signal processor (DSP), a state machine, or a programmable logic device. The processor 810 may also be a logical circuit, including a programmable gate array (PGA), such as a field programmable gate array (FPGA), or another type of circuit that includes discrete gate and/or transistor logic. The processor 810 may be a central processing unit (CPU), a graphics processing unit (GPU), or both. Additionally, any processor described herein may include multiple processors, parallel processors, or both. Multiple processors may be included in, or coupled to, a single device or multiple devices.

The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems. The term computing device should also be interpreted to include a collection or network of computing devices each including a processor or processors. Programs have software instructions performed by one or multiple processors that may be within the same computing device or which may be distributed across multiple computing devices.

The computer system 800 further includes a main memory 820 and a static memory 830, where memories in the computer system 800 communicate with each other and the processor 810 via a bus 808. Either or both of the main memory 820 and the static memory 830 may be considered representative examples of a memory of a controller, and store instructions used to implement some or all aspects of methods and processes described herein. Memories described herein are tangible storage mediums for storing data and executable software instructions and are non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The main memory 820 and the static memory 830 are articles of manufacture and/or machine components. The main memory 820 and the static memory 830 are computer-readable mediums from which data and executable software instructions can be read by a computer (e.g., the processor 810). Each of the main memory 820 and the static memory 830 may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, blu-ray disk, or any other form of storage medium known in the art. The memories may be volatile or non-volatile, secure and/or encrypted, unsecure and/or unencrypted.

"Memory" is an example of a computer-readable storage medium. Computer memory is any memory which is directly accessible to a processor. Examples of computer memory include, but are not limited to RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices.

As shown, the computer system 800 further includes a video display unit 850, such as a liquid crystal display (LCD), an organic light emitting diode (OLED), a flat panel display, a solid-state display, or a cathode ray tube (CRT), for example. Additionally, the computer system 800 includes an input device 860, such as a keyboard/virtual keyboard or touch-sensitive input screen or speech input with speech recognition, and a cursor control device 870, such as a mouse or touch-sensitive input screen or pad. The computer system 800 also optionally includes a disk drive unit 880, a signal generation device 890, such as a speaker or remote control, and/or a network interface device 840.

In an embodiment, as depicted in Fig. 8, the disk drive unit 880 includes a computer-readable medium 882 in which one or more sets of software instructions 884 (software) are embedded. The sets of software instructions 884 are read from the computer-readable medium 882 to be executed by the processor 810. Further, the software instructions 884, when executed by the processor 810, perform one or more steps of the methods and processes as described herein. In an embodiment, the software instructions 884 reside all or in part within the main memory 820, the static memory 830 and/or the processor 810 during execution by the computer system 800. Further, the computer-readable medium 882 may include software instructions 884 or receive and execute software instructions 884 responsive to a propagated signal, so that a device connected to a network 801 communicates voice, video or data over the network 801. The software instructions 884 may be transmitted or received over the network 801 via the network interface device 840.

In an embodiment, dedicated hardware implementations, such as application-specific integrated circuits (ASICs), field programmable gate arrays (FPGAs), programmable logic arrays and other hardware components, are constructed to implement one or more of the methods described herein. One or more embodiments described herein may implement functions using two or more specific interconnected hardware modules or devices with related control and data signals that can be communicated between and through the modules. Accordingly, the present disclosure encompasses software, firmware, and hardware implementations. Nothing in the present application should be interpreted as being implemented or implementable solely with software and not hardware such as a tangible non-transitory processor and/or memory.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

Accordingly, sensor-based treatment scheduling enables a shift from conventional hospital treatment by replacing conventional hospital treatment with unobtrusive vital signs sensor technologies, centered around the individual, to provide better information about the patient's general health condition. Such vital signs sensor technologies may reduce treatment costs by disease prevention and enhance the quality of life and, potentially, improved physiological data for physicians to analyze when attempting to diagnose a patient's general health condition. Algorithms described herein may estimate treatment readiness of patients and help hospitals scheduling surgeries/treatments to decrease surgery cancellations, increase same day surgery, estimate discharge readiness, start treatment whenever possible by indicating patient decompensation possibilities/patient physical status instead of guessing/basing estimations on relatively few metrics. The teachings herein describe employment of clinical decision support algorithms to define a patient's fitness to start a treatment based on unobtrusively measured vital signs, such as for fitness for surgery, fitness for intervention, and/or fitness for discharge. Based on these fitness estimations, patient selection for treatment may be improved.

Although sensor-based treatment scheduling has been described with reference to several exemplary embodiments, it is understood that the words that have been used are words of description and illustration, rather than words of limitation. Changes may be made within the purview of the appended claims, as presently stated and as amended, without departing from the scope and spirit of sensor-based treatment scheduling in its aspects. Although sensor-based treatment scheduling has been described with reference to particular means, materials and embodiments, sensor-based treatment scheduling is not intended to be limited to the particulars disclosed; rather sensor-based treatment scheduling extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. § 1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

## Claims

1. A central control system, comprising:

   a receiver configured to receive, from a plurality of wearable sensing systems according to claim 7, characteristic data determined to reflect health of wearers of the wearable sensing systems;
   a memory configured to store instructions; and
   a processor configured to execute the instructions, wherein, when the processor executes the instructions, the central control system is configured to:
   determine when the characteristic data indicates that any of the wearers of the wearable sensing systems is ready to be scheduled for a treatment, and
   initiate a process to schedule the treatment.

2. The central control system of claim 1, wherein the central control system is configured to receive the characteristic data over time and,
   when the processor executes the instructions, the central control system is further configured to: estimate a fitness level of a wearer of a wearable sensing system using a function that maps characteristic data received over time to a fitness level score, determine when the fitness level score for the wearer of the wearable sensing system exceeds a threshold, and initiate the process to schedule the treatment for the wearer of the wearable sensing system when the fitness level score for the wearer of the wearable sensing system exceeds the threshold.

3. The central control system of claim 2, wherein the function that maps characteristic data received over time to a fitness level score weights sums based on the characteristic data received over time, and at least one of the weighted sums comprises a weighted sum based on activity of the wearer of the wearable sensing system.

4. The central control system of claim 3, wherein:

   when the processor executes the instructions, the central control system is further configured to:
   add an internal factor index to the weighted sums for the wearer of the wearable sensing system, the internal factor index comprising a first value determined based on demographic and lifestyle characteristics of the wearer of the wearable sensing system; and
   add an external factor index to the weighted sums for the wearer of the wearable sensing system, the external factors index comprising a second value based on health characteristics of the wearer of the wearable sensing system.

**5.** The central control system of any of claims 2, 3 or 4, wherein:

when the processor executes the instructions, the central control system is further configured to: evaluate trends of the characteristic data over time; and

determine that the characteristic data indicates that the wearer of the wearable sensing system is ready to be scheduled for a treatment based on evaluations of trends of the characteristic data for the for the wearer of the wearable sensing system.

**6.** The central control system of claim 2, wherein the function that maps characteristic data received over time to a fitness level score comprises a pre-trained machine learning model which infers the fitness level score from the characteristic data.

**7.** A wearable sensing system, comprising:

a sensor configured to sense characteristic data determined to reflect health of a user of the sensing system;

a control system including a memory configured to store instructions and a processor configured to execute the instructions, wherein, when the processor executes the instructions, the control system is configured to determine when the characteristic data indicates that the user is ready to be scheduled for a treatment, and

a wireless communication unit configured to transmit a wireless notification to initiate a process to schedule the treatment.

**8.** The wearable sensing system of claim 7, wherein:

the characteristic data is sensed over time and,

when the processor executes the instructions, the control system is further configured to: estimate a fitness level of the user using a function that maps characteristic data received over time to a fitness level score, determine when the fitness level score for the user of the sensing system exceeds a threshold, and instruct the wireless communication unit to transmit the wireless notification when the fitness level score for the user of the sensing system exceeds the threshold.

**9.** The wearable sensing system of claim 8, wherein the function that maps characteristic data received over time to a fitness level score weights sums based on the characteristic data received over time and at least one of the weighted sums comprises a weighted sum based on activity of the user of the sensing system.

**10.** The wearable sensing system of claim 9, wherein, when the processor executes the instructions the control system is further configured to:

add an internal factor index to the weighted sums for the user of the sensing system, the internal factor index comprising a first value determined based on demographic and lifestyle characteristics of the user of the sensing system; and

add an external factor index to the weighted sums for the user of the sensing system, the external factors index comprising a second value based on health characteristics of the user of the sensing system.

**11.** The wearable sensing system of any of claims 8, 9 or 10, wherein:

when the processor executes the instructions, the control system is further configured to: evaluate trends of the characteristic data over time; and

determine that the characteristic data indicates that a user of the sensing system is ready to be scheduled for a treatment based on evaluations of trends of the characteristic data for the for the user of the sensing system.

**12.** An operation method for a central control system according to claim 1, comprising:

receiving from a plurality of wearable sensing systems according to claim 7, characteristic data determined to reflect health of wearers of the wearable sensing systems;

obtaining instructions by a processor from a memory;

determining, based on the processor executing the instructions, when the characteristic data indicates that any of the wearers of the wearable sensing systems is ready to be scheduled for a treatment, and

initiating a process to schedule the treatment.

13. The operation method of claim 12, wherein

the characteristic data is received over time and
the operation method further comprises:
estimating a fitness level of a wearer of the wearable sensing system using a function that maps characteristic data received over time to a fitness level score;
determining when the fitness level score for the wearer of the wearable sensing system exceeds a threshold, and initiating the process to schedule the treatment for the wearer of the wearable sensing system when the fitness level score for the wearer of the wearable sensing system exceeds the threshold.

14. An operation method of a wearable sensing system according to claim 7, comprising:

sensing, by a sensor of the wearable sensing system, characteristic data determined to reflect health of a wearer of the wearable sensing system;
obtaining instructions by a processor from a memory;
determining, by a control system that includes the processor and the memory, when the characteristic data indicates that the wearer is ready to be scheduled for a treatment, and
transmitting, by a wireless communication unit, a wireless notification to initiate a process to schedule the treatment.

15. The operation method of claim 14, wherein:

the characteristic data is sensed over time and,
the operation method further comprises
estimating a fitness level of the wearer of the wearable sensing system using a function that maps characteristic data received over time to a fitness level score;
determining when the fitness level score for the wearer of the wearable sensing system exceeds a threshold; and
instructing the wireless communication unit to transmit the wireless notification when the fitness level score for the wearer of the wearable sensing system exceed the threshold.

100

DISPLAY
180

CENTRAL
CONTROL
SYSTEM
140

CONTROLLER
150

PROCESSOR
152

MEMORY
151

SENSOR
110

SENSOR
120

SENSOR
130

101

FIG.1

**200**

**DISPLAY**
**280**

**SENSOR**
**210**

**CONTROLLER**
**250**

**PROCESSOR**
**252**

**MEMORY**
**251**

**WIRELESS COMMUNICATION UNIT**
**220**

**201**

**CENTRAL**
**CONTROL**
**SYSTEM**
**240**

# FIG.2

300

DISPLAY
180

SENSOR
210

CENTRAL
CONTROL
SYSTEM
140

301

CONTROLLER
250

CONTROLLER
150

PROCESSOR
252

MEMORY
251

PROCESSOR
152

MEMORY
151

# FIG.3

OBTAIN ISTRUCTIONS
S405

↓

RECEIVE CHARACTERISTIC DATA
S410

↓

ESTIMATE FITNESS LEVEL
S415

↓

SCHEDULE TREATMENT?
S420

NO → (back to S410)

↓ YES

INITIATE PROCESS
S425

**FIG.4A**

OBTAIN ISTRUCTIONS
S455

↓

SENSE CHARACTERISTIC DATA
S460

↓

ESTIMATE FITNESS LEVEL
S465

↓

SCHEDULE TREATMENT?
S470

NO → (back to S460)

↓ YES

TRANSMIT WIRELESS NOTIFICATION
S475

**FIG.4B**

581

PATIENT FITNESS

PATIENT DETERIORATED, BUT STILL STRONG ENOUGH FOR TREATMENT
TREATMENT CAN BE STARTED

THRESHOLD FITNESS FOR TREATMENT

PATIENT DETERIORATED TOO MUCH, TOO WEAK FOR TREATMENT
INTERVENTION NEEDED BEFORE TREATMENT CAN START

TIME

FIG.5

681

PATIENT IMPROVED ENOUGH TO COPE TREATMENT
SCHEDULE IN THIS PATIENT

THRESHOLD FITNESS FOR TREATMENT

PATIENT DID NOT IMPROVE ENOUGH TO COPE TREATMENT
DO NOT YET SCHEDULE IN THIS PATIENT

PATIENT FITNESS

TIME

FIG.6

S710       S720       S730       S740

| VITAL SIGNS SENSOR | ⇨ | FEATURE EXTRACTION | ⇨ | FITNESS PREDICTION MODEL | ⇨ | PATIENT FIT4TREATMENT SCORE |

# FIG.7

_800_

| | | |
|---|---|---|
| 810 PROCESSOR<br>INSTRUCTIONS | | 850 IMAGE/VIDEO DISPLAY |
| 820 MAIN MEMORY<br>INSTRUCTIONS | | 860 ALPHA-NUMERIC INPUT DEVICE |
| 830 STATIC MEMORY<br>INSTRUCTIONS | | 870 CURSOR CONTROL DEVICE |
| 840 DIGITAL INTERFACE | 808 | 880 DRIVE UNIT<br>882 COMPUTER READABLE MEDIUM<br>884 INSTRUCTIONS |
| 801 NETWORK | | 890 SIGNAL GENERATION DEVICE |

FIG.8

**EP 4 481 753 A1**

| | Europäisches Patentamt European Patent Office Office européen des brevets | **EUROPEAN SEARCH REPORT** | **Application Number** EP 23 19 7321 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/069370 A1 (RAO KRISHNA PRASAD AGARA VENKATESHA [IN] ET AL) 2 March 2023 (2023-03-02) * abstract * * paragraphs [0021], [0023], [0031], [0041], [0053], [0054], [0100], [0101], [0179] * ----- | 1-6,12, 13 | INV. G16H20/30 A61B5/00 G16H40/20 G16H50/30 ADD. A61B5/0205 A61B5/024 |
| X | US 2017/293729 A1 (MOVVA SATISH [US]) 12 October 2017 (2017-10-12) * abstract * * paragraphs [0076] - [0081] * ----- | 1-6,12, 13 | |

| | TECHNICAL FIELDS SEARCHED (IPC) |
| | G16H A61B |

The present search report has been drawn up for all claims

| Place of search | Date of completion of the search | Examiner |
|---|---|---|
| Munich | 11 March 2024 | Ricciardi, Maurizio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

Europäisches
Patentamt

European
Patent Office

Office européen
des brevets

**Application Number**

EP 23 19 7321

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

    see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

    1-6, 12, 13

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

1. claims: 1-6, 12, 13

   A central control system, comprising:a receiver configured to receive, from a plurality of wearable sensing systems according to claim 7, characteristic data determined to reflect health of wearers of the wearable sensing systems;a memory configured to store instructions; anda processor configured to execute the instructions, wherein, when the processor executes the instructions, the central control system is configured to:determine when the characteristic data indicates that any of the wearers of the wearable sensing systems is ready to be scheduled for a treatment, andinitiate a process to schedule the treatment.

   - - -

2. claims: 7-11, 14, 15

   A wearable sensing system, comprising:a sensor configured to sense characteristic data determined to reflect health of a user of the sensing system;a control system including a memory configured to store instructions and a processor configured to execute the instructions, wherein, when the processor executes the instructions, the control system is configured to determine when the characteristic data indicates that the user is ready to be scheduled for a treatment, anda wireless communication unit configured to transmit a wireless notification to initiate a process to schedule the treatment.

   - - -

**ANNEX TO THE EUROPEAN SEARCH REPORT
ON EUROPEAN PATENT APPLICATION NO.**

EP 23 19 7321

This annex lists the patent family members relating to the patent documents cited in  the above-mentioned European search report.
The members are as contained in the European Patent Office EDP file on
The European Patent Office is in no way liable for these particulars which are merely given for the purpose of information.

11-03-2024

| Patent document cited in search report | Publication date | Patent family member(s) | Publication date |
|---|---|---|---|
| US 2023069370 A1 | 02-03-2023 | CN 116848584 A | 03-10-2023 |
| | | EP 4374381 A1 | 29-05-2024 |
| | | US 2023069370 A1 | 02-03-2023 |
| | | WO 2023031728 A1 | 09-03-2023 |
| US 2017293729 A1 | 12-10-2017 | CA 2838232 A1 | 16-11-2014 |
| | | FR 3005767 A1 | 21-11-2014 |
| | | FR 3054693 A1 | 02-02-2018 |
| | | FR 3054694 A1 | 02-02-2018 |
| | | KR 20140135591 A | 26-11-2014 |
| | | KR 20180043765 A | 30-04-2018 |
| | | KR 20180103032 A | 18-09-2018 |
| | | KR 20190025881 A | 12-03-2019 |
| | | US 9734295 B1 | 15-08-2017 |
| | | US 2017293729 A1 | 12-10-2017 |
| | | US 2018025123 A1 | 25-01-2018 |

EPO FORM P0459

For more details about this annex : see Official Journal of the European Patent Office, No. 12/82